# EUROPÄISCHE PATENTSCHRIFT

(11) **EP 3 846 872 B1**
(45) Veröffentlichungstag und Bekanntmachung des Hinweises auf die Patenterteilung: **20.11.2024**
(21) Anmeldenummer: 19765985.7
(22) Anmeldetag: 05.09.2019
(51) Int. Cl.: A61M 1/16

(54) **KLIMATISIERUNGSVORRICHTUNG FÜR EIN BLUTBEHANDLUNGSGERÄT**
COOLING DEVICE FOR A BLOOD-TREATMENT APPARATUS.
DISPOSITIF DE REFROIDISSEMENT D'UN APPAREIL DE TRAITEMENT DU SANG

(30) Priorität: 05.09.2018 DE 102018121671
(43) Veröffentlichungstag der Anmeldung: 14.07.2021
(73) Patentinhaber: Fresenius Medical Care Deutschland GmbH, 61352 Bad Homburg (DE)
(72) Erfinder: WILD, Michael, 61352 Bad Homburg (DE); LECLERC, Andreas, 61231 Bad Nauheim (DE); RUSS, Carsten, 61118 Bad Vilbel (DE)
(74) Vertreter: Nordmeyer, Philipp Werner
(86) Internationale Anmeldenummer: PCT/EP2019/073749
(87) Internationale Veröffentlichungsnummer: WO 2020/049119

(56) Entgegenhaltungen:
- WO-A1-2017/156643
- DE-U1- 202017 104 462
- US-A1- 2004 211 718

## Beschreibung

### Technisches Gebiet

Die vorliegende Erfindung betrifft eine Medienversorgungseinrichtung zur Versorgung eines medizinischen Behandlungsgeräts mit einem Medium, beispielsweise zur Versorgung einer extrakorporalen Blutbehandlungsvorrichtung mit einem Dialysemedium.

### Stand der Technik

Immer wieder kommt es in Dialysekliniken zu Klagen von Patienten bei Dialysebehandlungen über den Raumluftkomfort. Die Patienten beklagen sich über zu hohe oder zu niedrige Raumlufttemperatur, zu hohe oder zu niedrige Luftfeuchtigkeit, zu schlechte Raumluft oder sie fühlen sich aufgrund von Zugerscheinungen unwohl. Diese Zugerscheinungen entstehen durch die eingebrachte zentrale Zuluft in den Raum über eine Klima- oder Lüftungsanlage.

In der Regel empfindet jeder Patient und auch das Personal den Raumluftzustand anders, insbesondere in Bezug auf die Temperatur. Dialysepatienten, die während der extrakorporalen Blutbehandlung oft sitzen oder liegen und sich kaum bewegen, frieren oft, wohingegen das Personal neben einer sitzenden und stehenden Tätigkeit wechselt und auch die Räumlichkeiten wechselt, somit aktiver und weniger kälteempfindlich ist.

Weiterhin ist für ein angenehmes Raumklima die Einspeisung von Frischluft erforderlich. Dazu werden neben der Klima- oder Lüftungsanlage oft einfach Fenster geöffnet, wobei hier die zuvor energetisch aufbereitete Zuluft verloren geht und es gleichzeitig zu Lärmbelästigungen von draußen kommen kann. Maßgeblich ist zudem auch wo sich der Patient im Raum befindet. Wenn sich der Behandlungsplatz zum Beispiel genau vor einem Fenster oder unter einem Luftauslass der Klimaanlage befindet, kann dies den Komfort negativ beeinflussen.

Insbesondere im Sommer, wenn aufgrund der Außentemperatur bereits ein Kühlbedarf besteht, ist die Problematik von Zugerscheinungen anzutreffen. Der Kühlbedarf ist darüber hinaus auch aufgrund der hohen inneren Wärmelasten, insbesondere der Wärmeerzeugung durch Personen und technischen Geräte, problematisch, zumal daraus erhöhte Volumenströme aus Klima- und Lüftungsanlagen resultieren und somit die bereits bestehenden Zugerscheinungen verstärken.

Herkömmliche Lösungen basierend auf einer zentralen Klimatisierung eines Raums mittels zentraler Gebäudetechnik bieten somit keine befriedigende Lösung für das unterschiedliche Wärme- und Kälteempfinden der Patienten sowie die unterschiedliche Wärmeerzeugung der jeweiligen Behandlungsgeräte, je nach Positionierung und Behandlungsphase. Folglich besteht ein Bedarf, die Klimatisierung von Behandlungsräumen zu verbessern und insbesondere für den jeweiligen Patienten individueller gestalten zu können.

### Darstellung der Erfindung

Ausgehend von dem bekannten Stand der Technik ist es eine Aufgabe der vorliegenden Erfindung, einen verbesserten Komfort für Patienten, die mit einem medizinischen Behandlungsgerät behandelt werden, bereitzustellen.

Die Aufgabe wird durch eine Medienversorgungseinrichtung mit den Merkmalen des Anspruchs 1 gelöst. Vorteilhafte Weiterbildungen ergeben sich aus den Unteransprüchen, der vorliegenden Beschreibung sowie den Figuren.

Entsprechend wird eine Medienversorgungseinrichtung zur Versorgung eines medizinischen Behandlungsgeräts mit einem Medium, insbesondere zur Versorgung einer extrakorporalen Blutbehandlungsvorrichtung mit einem Dialysemedium, vorgeschlagen. Erfindungsgemäß ist eine Klimatisierungsvorrichtung zur Klimatisierung eines an die Medienversorgungseinrichtung angeschlossenen medizinischen Behandlungsgeräts vorgesehen.

Durch das Bereitstellen der Klimatisierungsvorrichtung in der Medienversorgungseinrichtung kann eine Klimatisierung des medizinischen Behandlungsgeräts und insbesondere der extrakorporalen Blutbehandlungsvorrichtung erreicht werden, wodurch Wärmelasten, die bei der Verwendung herkömmlicher Medienversorgungseinrichtungen in die Umgebung und insbesondere in die Raumluft des Behandlungsraums abgegeben werden, zumindest teilweise aus dem Behandlungsraum abgeleitet werden. Damit kann die für die Raumluft des Behandlungsraums aufzuwendende Klimatisierungsleistung reduziert werden, so dass das Auftreten von Zuglufterscheinungen reduziert werden kann.

In der WO 2017/156643 A1 wird ein Modul offenbart, welches ein Kühlelement und ein Heizelement umfasst, um eine für Lösungswasser erforderliche Temperierung bereitzustellen. Das Modul ist in einer Vorrichtung zum Herstellen einer Dialysierflüssigkeit integriert.

Eine interne Kühlung mittels eines Lüfters und einer Leitung ist weiterhin aus der US 2004/211718 A1 bekannt, wobei die Kühlung innerhalb einer Vorrichtung zum Herstellen einer Dialysatlösung angeordnet ist, um einen entsprechenden Behälter zu kühlen.

Weiterhin sind verschiedene Einheiten eines Wärmetauschers aus der DE 20 2017 104 462 U1 bekannt.

Durch die Anordnung der Klimatisierungsvorrichtung in der Medienversorgungseinrichtung besteht eine räumliche Nähe zwischen dem medizinischen Behandlungsgerät und der Klimatisierungsvorrichtung, da das medizinische Behandlungsgerät stets in der unmittelbaren Umgebung der Medienversorgungseinrichtung aufgestellt wird, um eine Versorgung des medizinischen Behandlungsgeräts mit Medien zu erreichen.

Durch die räumliche Nähe zwischen der Medienversorgungseinrichtung und dem medizinischen Behandlungsgerät kann die Klimatisierungsvorrichtung aber auf die direkte Umgebung des medizinischen Behandlungsgeräts wirken und so die von dem medizinischen Behandlungsgerät abgegebenen Wärmelasten zumindest teilweise abführen. Diese Abführung der Wärmelasten aus der direkten Umgebung des medizinischen Behandlungsgeräts hat den Effekt, dass die Wärmelasten entsprechend nicht oder nur in einem reduzierten Umfang in die übrige Raumluft, also quasi die entferntere Umgebung des medizinischen Behandlungsgeräts, eingetragen werden. Damit wird das von dem zu behandelnden Patienten empfundene Raumklima nicht oder nur noch in geringerem Maße von den durch das medizinische Behandlungsgerät eingetragenen Wärmelasten beeinflusst.

Mit anderen Worten findet eine lokale Klimatisierung in der Umgebung der Medienversorgungseinrichtung und damit gleichzeitig in der Umgebung des medizinischen Behandlungsgeräts statt.

Bei dem medizinischen Behandlungsgerät kann es sich beispielsweise um eine extrakorporale Blutbehandlungsvorrichtung wie beispielsweise eine Apheresevorrichtung oder eine Dialysevorrichtung handeln, welche beispielsweise als Hämodialysevorrichtung, als Hämofiltrationsvorrichtung, oder als Hämodiafiltrationsvorrichtung ausgebildet sein kann. Dialysevorrichtungen werden üblicher Weise an Medienversorgungseinrichtungen angeschlossen, um eine Versorgung beispielsweise mit Dialysewasser, Dialysekonzentrat und/oder Strom zu erreichen. Dabei werden die Dialysevorrichtungen in der unmittelbaren Nähe der Medienversorgungseinrichtung platziert, um die Längen der Anschlussleitungen und Anschlusskabel zu reduzieren und um eine Arbeitsplatzsicherheit durch die Abwesenheit von durch aus Leitungen bestehenden Stolperfallen bereit zu stellen. Es besteht daher eine räumliche Nähe zwischen der Dialysevorrichtung und der Medienversorgungseinrichtung.

Bevorzugt umfasst die Medienversorgungeinrichtung einen Anschluss zum Anschließen des medizinischen Behandlungsgeräts zum Zuführen eines Mediums zu dem medizinischen Behandlungsgerät und/oder einen Ablaufanschluss zum Abführen eines Mediums von dem medizinischen Behandlungsgerät.

Durch die Medienversorgungseinrichtung, die auch Medienversorgungspaneel oder Media Supply Panel genannt wird, wird somit gleichzeitig eine Klimatisierung als auch eine erforderliche Fluidversorgung für das medizinische Behandlungsgerät bereitgestellt.

Die Medienversorgungseinrichtung kann mit einem eigenen Abdeckungspaneel ausgebildet sein, so dass sie in eine entsprechende Ausnehmung in einer Wand oder in einer Wandvorsatzschale eingebaut werden kann. Das Abdeckungspaneel überdeckt dabei die Ausnehmung in der Wand oder Wandvorsatzschale, so dass mittels des Abdeckungspaneels zum einen eine geschlossene Wandkontur ausgebildet werden kann, was aus hygienischen Gründen wünschenswert ist und die einzelnen Anschlüsse und Auslässe der Medienversorgungseinrichtung befinden sich auch in einer vorgegebenen und bevorzugten Anordnung zueinander. Zum anderen kann die Ausnehmung in der Wand oder Wandvorsatzschale so ästhetisch ansprechend verschlossen werden.

Die Medienversorgungseinrichtung kann auch mit einem eigenen Gehäuse mit Abdeckungspaneel ausgebildet sein, in welchem sich Komponenten der Medienversorgungseinrichtung befinden und welches auf einer Wandfläche befestigt werden kann. Damit kann eine Montage der Medienversorgungseinrichtung auch auf Wandabschnitten vorgenommen werden, welche nur eine geringe Einbautiefe ermöglichen.

Die Medienversorgungseinrichtung kann in einer weiteren Alternative auch ohne ein eigenes Abdeckungspaneel bereitgestellt werden, so dass die einzelnen Anschlüsse und Auslässe dann in einer vorhandenen oder bereitzustellenden Abdeckung aufgenommen werden können. Auf diese Weise kann eine bereits vorhandene Anmutung eines Wandbereichs besser aufrechterhalten bleiben beziehungsweise die Anschlüsse und Auslässe der Medienversorgungseinrichtung können in eine bereits vorhandene Infrastruktur einfacher integriert werden.

Um eine verbesserte Klimatisierung bereitzustellen und diese weitestgehend flexibel und auf die jeweiligen Bedürfnisse anpassbar zu gestalten, kann die Klimatisierungsvorrichtung bevorzugt einen Temperatursensor zum Messen der Temperatur und/oder der Umgebungstemperatur des medizinischen Behandlungsgeräts aufweisen und eine mit dem Temperatursensor kommunikativ verbundene Steuer-/Regeleinheit kann dazu eingerichtet sein, die Klimatisierungsleistung der Klimatisierungsvorrichtung basierend auf einem Wert des Temperatursensors zu steuern/regeln, bevorzugt über einen Volumenstrom eines jeweiligen mit der Steuer-/Regeleinheit verbundenen Ventilators.

Beispielsweise können in der Steuer-/Regeleinheit Soll-Werte hinterlegt sein, welche Zustände für das medizinische Behandlungsgerät festlegen, und wobei Ist-Werte von dem Temperatursensor empfangen werden. Bei Abweichung dieser Werte, insbesondere wenn der Ist-Wert den entsprechenden Soll-Wert übersteigt, regelt die Steuer-/Regeleinheit beispielsweise einen Volumenstrom des durch den Ventilator bereitgestellten Luftstroms im Abluftkanal und, bei entsprechender Ausgestaltung der Medienversorgungseinrichtung, ebenfalls im Zuluftkanal, beispielsweise über die Drehzahl des jeweiligen Ventilators und bevorzugt mit einem entsprechenden Regelwert oder Stellwert. So kann eine zu hohe Temperatur zu einer Erhöhung des Luftstroms führen während der Luftstrom bei einer zu niedrigen Temperatur verringert wird. In einer einfachen Ausgestaltung kann jedoch auch vorgesehen sein, dass der Sensor nur beim Übersteigen eines Schwellenwerts ein Signal abgibt, wobei die Steuer-/Regeleinheit den jeweiligen Luftstrom beziehungsweise die jeweilige Drehzahl des Ventilators auf einen vorgegebenen Wert erhöht.

Der Temperatursensor kann dabei zur Befestigung an dem medizinischen Behandlungsgerät und zur drahtlosen Kommunikation mit der Steuer-/Regeleinheit eingerichtet sein, bevorzugt über Funk. Entsprechend kann der Temperatursensor einfach an das medizinische Behandlungsgerät angebracht werden und es können Messwerte beispielsweise über eine drahtlose Kopplung wie NFC oder Bluetooth an die Steuer-/Regeleinheit übermittelt werden.

Alternativ dazu kann der Temperatursensor auch über eine Kabelverbindung mit der Steuer-/Regeleinheit verbunden sein. Dazu kann der Temperatursensor beispielsweise in Form eines Wurfthermometers ausgebildet sein, welches beispielsweise einen in einem Sensorgehäuse angeordneten Temperatursensor aufweist, der dann über ein flexibles Kabel an die Steuer-/Regeleinheit angebunden sein kann. Ein solches Wurfthermometer kann dann über einen Teil des medizinischen Behandlungsgeräts gehängt werden, so dass es dann die im Bereich des eigentlichen Temperatursensors gewonnenen Temperaturwerte über die Kabelverbindung an die Steuer-/Regeleinheit übergibt.

Bei Verwendung eines kabelbehafteten Temperatursensors kann an der Medienversorgungseinrichtung ein entsprechender Anschluss, beispielsweise in Form einer Steckerbuchse, vorgesehen sein, an welchen ein dazu korrespondierender Anschluss des Temperatursensors beziehungsweise dessen Kabel angeschlossen werden kann, beispielsweise durch einen in die Steckerbuchse einsteckbaren Stecker. Die Weiterleitung der Temperaturwerte an die Steuer-/Regeleinheit findet dann entsprechend durch die Medienversorgungseinrichtung statt.

Durch die Verwendung eines kabelgebundenen Temperatursensors kann auf die Verwendung von Energiespeichern, beispielsweise Batterien, in dem Temperatursensor zur Energieversorgung einer drahtlosen Kommunikationsverbindung verzichtet werden, so dass sich auf diese Weise eine strahlungsarme, zuverlässige und kostengünstige Temperaturbestimmung an dem medizinischen Behandlungsgerät erreichen lässt. Das zusätzlich verwendete Kabel ist dabei nicht notwendiger Weise hinderlich, da das medizinische Behandlungsgerät ohnehin über Kabel und Leitungen an der Medienversorgungseinrichtung angeschlossen ist und das Vorliegen eines weiteren Kabels daher nicht ins Gewicht fällt.

Durch das Positionieren des Temperatursensors an oder auf dem medizinischen Behandlungsgerät können weiterhin eine optimale Anordnung beziehungsweise Ausrichtung des medizinischen Behandlungsgeräts in Bezug auf den jeweiligen Luftkanal und eine zuverlässige Direktmessung erfolgen. Alternativ oder zusätzlich kann jedoch ebenfalls ein Temperatursensor oder Temperaturfühler vorgesehen sein, welche mittels eines Kabels kommunikativ mit der Steuer-/Regeleinheit verbunden ist.

Obwohl der Temperatursensor einen zuverlässigen Messwert für die erforderliche Klimatisierung darstellt, kann alternativ oder zusätzlich ebenfalls vorgesehen sein, dass die Steuer-/Regeleinheit dazu eingerichtet ist, einen Betriebsparameter des medizinischen Behandlungsgeräts zu empfangen und die Klimatisierungsleistung basierend auf dem Betriebsparameter zu steuern/regeln, wobei der Betriebsparameter bevorzugt mit einem bestimmten Betriebsmodus übereinstimmt.

Somit kann bereits in einem frühen Stadium und unabhängig von der gemessenen Temperatur eine Kühlung bereitgestellt werden, um beispielsweise eine anstehende Änderung zu antizipieren. Beispielsweise kann es bei einer Heiß-Desinfektion des medizinischen Behandlungsgeräts nach der Behandlung zu hoher Wärmeabstrahlung kommen, welche den Raum erheblich aufheizt. Der Betriebsparameter kann in diesem Fall für eine solche Desinfektionsphase kennzeichnend sein. Entsprechend kann die Steuer-/Regeleinheit beim Empfangen eines solchen Betriebsparameters mit einer entsprechenden Stellgröße den Ventilator ansprechen und somit die Wärme über den Luftstrom aus dem Raum bringen.

Die Klimatisierungsvorrichtung umfasst einen Abluftkanal zum Abführen von Luft aus der Umgebung des medizinischen Behandlungsgeräts, wobei ein erster Ventilator zum Abführen von Luft aus der Umgebung des medizinischen Behandlungsgeräts in den Abluftkanal hinein vorgesehen ist. Durch das Bereitstellen des Abluftkanals in der Klimatisierungsvorrichtung der Medienversorgungseinrichtung kann entsprechend Luft aus der direkten Umgebung der Medienversorgungseinrichtung und damit aus der direkten Umgebung des medizinischen Behandlungsgeräts abgezogen werden und auf diese Weise die anfallenden Wärmelasten zumindest teilweise abgeführt werden.

Hierbei wird wieder die ohnehin vorliegende räumliche Nähe zwischen dem medizinischen Behandlungsgerät und der Medienversorgungseinrichtung dazu verwendet, gezielt die Wärmelasten des medizinischen Behandlungsgeräts abzuführen. Es versteht sich aber, dass bei einem Abziehen von Umgebungsluft auch Luftvolumina aus der Raumluft abgezogen werden können, die nicht durch das medizinische Behandlungsgerät beeinflusst waren. Die thermische Kopplung zwischen der Medienversorgungseinrichtung und dem medizinischen Behandlungsgerät ist daher nicht ausschließlich, aber es kann dennoch die durch das medizinische Behandlungsgerät beeinflusste Umgebungsluft in einem solchen Umfang abgezogen werden, dass ein spürbarer Effekt auf die übrige Raumluft zu verzeichnen ist.

Durch das Bereitstellen des Abluftkanals und des Ventilators wird somit ermöglicht, dass Wärme von dem medizinischen Behandlungsgerät mittels eines Luftstroms weggeführt werden kann. Beispielsweise kann der Luftstrom dabei entweder ausgehend von oder zumindest teilweise entlang des medizinischen Behandlungsgeräts geführt werden, wobei der Abluftkanal dabei bevorzugt derart konfiguriert ist, dass der Luftstrom aus dem Raum, in dem sich das medizinische Behandlungsgerät befindet, nach außen abgeführt wird oder einem Sammelkanal zugeführt wird. Die von dem medizinischen Behandlungsgerät erzeugte Wärme wird somit aus dem Behandlungsbereich abgeführt. Damit kann der Wärmeeintrag in den Behandlungsraum reduziert werden und damit der Klimatisierungsbedarf in dem Behandlungsraum reduziert werden. Auf diese Weise kann das Raumklima verbessert werden, weil die thermischen Lasten, die durch den Betrieb des medizinischen Behandlungsgeräts in den Behandlungsraum eingetragen werden deutlich reduziert werden können.

Folglich wird durch die vorgeschlagene Medienversorgungseinrichtung eine teilweise Entkopplung der Kühlung des medizinischen Behandlungsgeräts von der Raumklimatisierung des Behandlungsraums erreicht. Für den Patienten ergibt sich damit eine mögliche Reduktion der Belüftung beziehungsweise der Kühlung, sodass eventuelle Zugerscheinungen ebenfalls reduziert werden können.

Gleichzeitig befindet sich ein Patient regelmäßig in der Nähe eines entsprechenden medizinischen Behandlungsgeräts. Eine oft als unangenehm empfundene Wärmestrahlung durch die im Normalbetrieb des medizinischen Behandlungsgeräts erzeugte Wärme wird somit ebenfalls reduziert.

Der erste Ventilator kann beispielsweise vor, während und/oder nach einer Behandlung aktiv sein, um den Luftstrom und somit die Kühlwirkung entsprechend bereitzustellen. Beispielsweise kann ein vor der Behandlung bereitgestellte Luftstrom bewirken, dass eine Vorkühlung des medizinischen Behandlungsgeräts bereitgestellt wird und somit, je nach Dauer der Behandlung, auf einen Luftstrom und eine entsprechende Aktivierung des Ventilators während der Behandlung verzichtet werden kann. Entsprechend kann auch eine Nachkühlung vorgesehen sein, beispielsweise für den Fall, bei dem eine Wärmeerzeugung insbesondere am Ende der Behandlung stattfindet. Somit werden während der Behandlung ebenfalls Geräusche reduziert, welche für den Patienten als unangenehm empfunden werden können.

Der Ventilator kann dabei entweder kontinuierlich angeschaltet oder es können Zeitabschnitte für den Betrieb des Ventilators vorgegeben sein, um beispielsweise den Energieverbrauch zu reduzieren. Um das Volumen der aus dem Behandlungsraum abgeführten Luft auszugleichen und damit Druckunterschiede zu vermeiden, kann weiterhin vorgesehen sein, dass eine zentrale Zuluft unabhängig von der Klimatisierungsvorrichtung kontinuierlich oder je nach zentraler Steuerung/Regelung in den Raum eingebracht wird, beispielsweise über eine konventionelle Lüftung.

Um eine optimale Kühlwirkung bereitzustellen, wird das medizinische Behandlungsgerät bevorzugt auf einer geeigneten, vorgesehenen Stellfläche positioniert. Entsprechend kann eine gekennzeichnete Fläche zum Positionieren des medizinischen Behandlungsgeräts vorgesehen sein, wobei der Abluftkanal und/oder der erste Ventilator dazu konfiguriert sind, Luft von der gekennzeichneten Stellfläche abzuführen und in den Abluftkanal zu fördern.

Beispielsweise kann im Behandlungsraum eine Markierung vorgesehen sein, welche die entsprechende Stellfläche und somit die vorgesehene Position des medizinischen Behandlungsgeräts anzeigt. Das medizinische Behandlungsgerät kann beispielsweise vor dem Abluftkanal und ausgerichtet an dessen Luftstrom positioniert werden. Somit wird sichergestellt, dass die Abluft dezentral und direkt am Behandlungsplatz abgezogen wird, um eine lokale Ansammlung der von der Blutbehandlungsvorrichtung und dem Patienten erzeugten Wärme zu verhindern.

Bevorzugt umfasst die Klimatisierungsvorrichtung einen Zuluftkanal zum Zuführen von Luft in die Umgebung des medizinischen Behandlungsgeräts, wobei insbesondere ein zweiter Ventilator zum Zuführen von Luft in die Umgebung des medizinischen Behandlungsgeräts vorgesehen ist.

Somit kann auf einfache Weise Zuluft entsprechend der Abluft in den Raum eingebracht werden, sodass die bereitgestellte Klimatisierung beziehungsweise Kühlwirkung keine Druckunterschiede beziehungsweise einen Unterdruck erzeugt. Damit kann auch ein noch besser gerichteter Luftstrom bereitgestellt werden, der eine Kühlung des medizinischen Behandlungsgeräts ermöglicht.

Ein Entstehen von Unterdruck in einem Behandlungsraum zu vermeiden bringt als weiteren Vorteil mit sich, dass ungewollte Sogwirkungen vermieden werden könne. Von solchen Sogwirkungen können Nachteile oder sogar Gefahren ausgehen: Türen können zufallen oder es kann eine größere Kraftanstrengung notwendig sein, um diese zu öffnen. Gerade bei geschwächten Personen oder bei eventuell auftreten Notfällen können solche Sogwirkungen verheerende Nachwirkungen haben. Insofern ist es besonders wünschenswert, diese zu vermeiden. Dazu möchte man Druckunterschiede vermeiden. Darüber hinaus ist es bevorzugt, einen relativen Unterdruck in einem Behandlungsraum zu vermeiden, weil ein Unterdruck dazu führen könnte, dass Keime wie z.B. Bakterien und Viren durch den Druckunterschied aus dem Umfeld in einen Behandlungsraum gedrückt werden. Daher ist es aus hygienischer Sicht bevorzugt, einen Unterdruck in einem Raum zu vermeiden.

Weiterhin kann durch das Einbringen von Zuluft qualitativ hochwertige Luft in den Raum eingebracht werden, welche sich beispielsweise an der Lufttemperatur, der CO₂-Konzentration und der relativen Luftfeuchtigkeit bemessen lässt. Um das eventuelle Einströmen von unerwünschten Partikeln oder Verunreinigungen zu verhindern, kann am oder im Zuluftkanal ein Filter vorgesehen sein.

Das Abführen von Abluft über den Abluftkanal und/oder das Einbringen von Zuluft über den Zuluftkanal hat weiterhin den Vorteil, dass Fenster in einem Behandlungsraum vorwiegend geschlossen bleiben können und somit einerseits die sonst empfundene Zugerscheinungen vermieden und andererseits Energie gespart werden kann.

Bevorzugt ist der jeweilige Ventilator in dem jeweiligen Luftkanal angeordnet und ist der der erste Ventilator bevorzugt an einem ersten Ende angeordnet.

Durch eine solche Anordnung kann die Medienversorgungseinrichtung relativ kompakt gehalten und kann weiterhin der Wirkungsgrad des Ventilators zum Bereitstellen des Luftstroms erhöht werden. Die Anordnung des ersten Ventilators an dem ersten Ende hat weiterhin den Vorteil, dass Luft in den Abluftkanal gezogen beziehungsweise angesaugt und die entsprechende Sogwirkung nicht durch die Länge des Abluftkanals verringert wird.

Bevorzugt ist eine Markierung zur Kennzeichnung eines Aufstellplatzes für das medizinische Behandlungsgerät im Wirkungsbereich der Klimatisierungsvorrichtung vorgesehen.

Entsprechend kann das medizinische Behandlungsgerät auch beispielsweise mobil (i.e. auf Rollen) ausgebildet sein und in einen Behandlungsraum auf einer mit der Markierung gekennzeichneten Fläche positioniert werden, sodass das medizinische Behandlungsgerät je nach Behandlungsbedarf und gewünschtem Behandlungsort für einen Patienten einsetzbar ist und lediglich an die Medienversorgungseinrichtung angekoppelt werden muss. Dadurch kann beispielsweise ein für die Behandlung erforderliches Fluid eingespeist werden während gleichzeitig eine dezentrale Klimatisierung bereitgestellt und eine erwünschte, lokale Kühlung erfolgt.

Weiterhin können durch eine Ausgestaltung mit mindestens einem Ablaufanschluss beispielsweise Abbauprodukte oder bereits verwendete Fluide abgeführt und entweder in der Medienversorgungseinrichtung gespeichert oder über eine entsprechende Fluidkopplung über ein zentrales Fluidsystem abgeführt werden. Die Medienversorgungseinrichtung ist somit für eine Vielzahl von Verwendungen einsetzbar.

Bevorzugt ist das Fluid eine medizinische Flüssigkeit, ein medizinisches Gas, ein Konzentrat, ein Permeat, eine Dialysierflüssigkeit, ein Dialysekonzentrat oder Wasser. Mit anderen Worten können jegliche für eine Behandlung eines Patienten und beispielsweise für eine Dialyse erforderliche Flüssigkeiten durch die Medienversorgungseinrichtung bereitgestellt werden. Ebenfalls können dabei medizinische Gase bereitgestellt werden, beispielsweise medizinischer Sauerstoff oder gasförmige Analgetika, sodass die Medienversorgungseinrichtung für verschiedene Patientengruppen und verschiedene Behandlungen eingesetzt werden kann.

Für Räume, in denen mehrere medizinische Behandlungsgeräte vorgesehen sind, ist die Medienversorgungseinrichtung dazu eingerichtet, mindestens zwei medizinische Behandlungsgeräte mit Medien zu versorgen und für jedes der medizinischen Behandlungsgeräte kann ein separater Zuluftkanal und/oder ein separater Abluftkanal zugeordnet sein, wobei der jeweilige Zuluftkanal oder der jeweilige Abluftkanal bevorzugt mit einem zentralen Abluftkanal oder einem zentralen Zuluftkanal gekoppelt sind.

Dabei sind bevorzugt mindestens zwei Anschlüsse zur Versorgung von mindestens zwei medizinischen Behandlungsgeräten mit mindestens einem Medium und/oder mindestens zwei Ablaufanschlüsse zum Abführen von mindestens einem Medium vorgesehen, um mindestens zwei medizinische Behandlungsgeräte mit Medien zu versorgen.

Entsprechend kann für jedes medizinische Behandlungsgerät zumindest einen Fluidanschluss vorgesehen sein, sodass beispielsweise zwei Dialysevorrichtungen mit beispielsweise einem Dialysat oder Dialysierflüssigkeit versorgt werden können. Bevorzugt sind ebenfalls zumindest zwei Ablaufanschlüsse vorgesehen, sodass die jeweilige Dialysevorrichtung ebenfalls beispielsweise ein Retentat oder verwendetes Dialysat entsorgen kann.

Für Fälle, bei denen mehrere medizinische Behandlungsgeräte in einem Behandlungsraum vorgesehen sind, kann weiterhin vorgesehen sein, dass für jedes medizinische Behandlungsgerät ein separater Zuluftkanal und/oder ein separater Abluftkanal vorgesehen ist, wobei der jeweilige Zuluftkanal und/oder der jeweilige Abluftkanal bevorzugt mit einem zentralen Abluftkanal oder einem zentralen Zuluftkanal gekoppelt sind.

Bevorzugt sind dabei für jedes medizinische Behandlungsgerät sowohl ein jeweiliger Zuluftkanal als auch ein jeweiliger Abluftkanal vorgesehen, sodass eine verbesserte, separate und individuelle Klimatisierung bereitgestellt wird.

Insbesondere in Dialyseräumen sind regelmäßig mehrere Dialysevorrichtungen zur gleichzeitigen Behandlung mehrerer Patienten vorhanden. Um die von den jeweiligen Geräten erzeugte Wärme effizient zu verringern, sind somit für jede Dialysevorrichtung separate Luftkanäle vorgesehen. Entsprechend kann eine Belüftung oder Klimatisierung mittels vorhandener, zentraler Gebäudetechnik reduziert beziehungsweise muss die zusätzlich erzeugte Wärme nicht davon ausgeglichen werden. In dem jeweiligen Raum entstehen somit keine unerwünschten Luftströmungen, sodass die Behandlung eines jeweiligen Patienten kein unangenehmes Raumklima für den jeweiligen Patienten oder die anderen Patienten bereitstellt. Diese Wirkung kann auch dadurch erreicht werden, dass ein Zuluftkanal und/oder ein Abluftkanal für beispielsweise zwei Dialysegeräte vorgesehen sind, wobei diese entsprechend an gegenüberliegenden Seiten der Dialysevorrichtungen angeordnet sind oder die

Dialysevorrichtungen in entgegengesetzten Richtungen orientiert sind, beispielsweise, wenn die Wärme erzeugenden Komponenten vorwiegend an einer Seite der jeweiligen Dialysevorrichtung angeordnet sind.

Die Ausgestaltung für mehrere Dialysevorrichtungen hat den Vorteil, dass sowohl bei einer Behandlung als auch bei mehreren Behandlungen stets eine gewünschte lokale Klimatisierung erfolgen kann, ohne dass dabei weitere Medienversorgungseinrichtungen erforderlich sind. Mit anderen Worten können auch bei einer beliebigen Zuschaltung von Dialysevorrichtungen eine Klimatisierung und eine Fluidversorgung erfolgen, sodass ein hohes Maß an Flexibilität bereitgestellt wird.

Bevorzugt weist die Klimatisierungsvorrichtung eine Drosselklappe zum Mischen von Zuluft und Abluft auf. Entsprechend besteht die Möglichkeit, dass die Zuluft mit der Abluft gemischt wird, bevor es in den vorgesehenen Raum eingebracht wird. Dies hat insbesondere den Vorteil, dass die einzubringende Luft zumindest teilweise temperiert oder vorgewärmt werden kann, beispielsweise wenn für die Kühlwirkung und/oder den Patienten zu kühle Außenluft eingespeist wird. Somit kann ebenfalls Energie gespart werden. Weiterhin kann umgekehrt die Abluft vorgekühlt werden, beispielsweise um zu verhindern, dass die abgeführte Luft eine zu hohe Temperatur erreicht, welche beispielsweise für benachbarte Systeme oder sonstige Komponenten eines medizinischen Behandlungsgeräts schädlich sein kann.

Bevorzugt kann die Klimatisierungsvorrichtung mit dem medizinischen Behandlungsgerät direkt thermisch koppelbar sein, bevorzugt über ein Wärmeleitelement. Beispielsweise kann das Wärmeleitelement an einem ersten Ende des Abluftkanals vorgesehen sein. Die thermische Kopplung kann somit über einen bereitgestellten Luftstrom erfolgen, wobei eine Wärmeübertragung ebenfalls über das vorgesehene Wärmeleitelement erfolgen kann, sodass die Wärmeabfuhr weiter verbessert werden kann.

Entsprechend kann das Wärmeleitelement eine Fläche bilden, welche Wärme aufnimmt und diese an den Abluftkanal und über den Luftstrom wieder abgibt. Weiterhin kann das Wärmeleitelement derart geformt sein, dass der Luftstrom für eine vorgegebene Fläche des medizinischen Behandlungsgeräts bereitgestellt wird. Beispielsweise kann das Wärmeleitelement eine der Fläche entsprechende Form, beispielsweise eine zylindrische oder kegelförmige Form aufweisen.

Um den Wirkungsgrad der Klimatisierung und den Wärmetransport weiter zu verbessern, ist der jeweilige Luftkanal bevorzugt zumindest teilweise mit einem thermisch isolierenden Material ausgekleidet. Somit wird sichergestellt, dass die Wärme nicht versehentlich an die Umgebung beziehungsweise an den jeweiligen Raum abgegeben wird.

Wenn die Medienversorgungseinrichtung einen Zuluftkanal aufweist, kann durch eine entsprechende Auskleidung weiterhin verhindert werden, dass die kühle Zuluft aufgewärmt wird bevor es in den entsprechenden Raum eingegeben wird. Obwohl der jeweilige Kanal bevorzugt der Länge nach und umlaufend mit dem thermisch isolierenden Material ausgekleidet ist, kann ebenfalls vorgesehen sein, dass lediglich die in den Raum mündenden Enden oder Fluidkopplungen thermisch isoliert sind, wobei der restliche Luftkanal beispielsweise von dem Raum getrennt und von Außenluft oder Umgebungsluft umgeben ist.

Bevorzugt weist die Klimatisierungsvorrichtung einen Schalldämpfer auf, wobei der Schalldämpfer bevorzugt an einem Zuluftkanal und/oder einem Abluftkanal und/oder einem Ventilator angebracht ist. Um zu verhindern, dass die Kühlwirkung unangenehme Geräusche verursacht, kann mit dem Schalldämpfer eine Reduzierung der Geräuschbelastung erreicht werden. Beispielsweise kann der Ventilator in dem jeweiligen Luftkanal angeordnet sein, wobei der Schalldämpfer an dem Ventilator oder benachbart zum Ventilator an dem Luftkanal befestigt ist.

Für eine genauere Regelung der Klimatisierung kann die Medienversorgungseinrichtung weiterhin einen Feuchtigkeitssensor, einen CO₂-Sensor, und/oder einen Durchflusssensor aufweisen, wobei der Sensor in einem jeweiligen Luftkanal angeordnet und mit der Steuer-/Regeleinheit kommunikativ verbunden ist. Die Steuer-/Regeleinheit ist dabei dazu eingerichtet, den jeweiligen Volumenstrom basierend auf dem Wert des Sensors zu steuern/regeln.

Dies hat insbesondere den Vorteil, dass die einzubringende Luft an das erforderliche Raumklima angepasst werden kann. Beispielsweise kann die Steuer-/Regeleinheit bei einer gemessenen zu hohe CO₂-Konzentration veranlassen, dass der Volumenstrom im Abluftkanal erhöht wird, sodass die im Raum vorhandene Luft von dem Behandlungsplatz beziehungsweise von dem medizinischen Behandlungsgerät abgeführt wird, wobei gleichzeitig eine Erhöhung des Volumenstroms im Zuluftkanal eine Einspeisung mit frischer Luft, beispielsweise Außenluft, bewirkt. Entsprechend kann eine Ausgestaltung mit nur einem Abluftkanal bereits zur erwünschten Verringerung der CO₂-Konzentration führen, wobei eine Ausgestaltung mit einem Zuluftkanal die synergetische Wirkung hat, dass gleichzeitig ein Einströmen von CO₂-armer Luft bereitgestellt wird.

Durch den Durchflusssensor kann weiterhin sichergestellt werden, dass eine ausreichende Frischluftversorgung bereitgestellt ist, beispielsweise durch das Erhöhen des Volumenstroms im Zuluftkanal, wenn der empfangene Messwert zu niedrig ist. Ein zu hoher Messwert kann jedoch für den Patienten als unangenehm empfunden werden, sodass der Volumenstrom in einem solchen Fall entsprechend verringert werden kann.

Um die Eigenschaften der zugeführten Luft weiter flexibel zu regeln, kann weiterhin vorgesehen sein, dass mindestens ein Luftkanal, bevorzugt jeder Luftkanal, ein Heizelement, ein Kühlelement, ein Befeuchtungselement, und/oder ein Entfeuchtungselement aufweist, welches mit der Steuer-/Regeleinheit kommunikativ verbunden ist, wobei die Steuer-/Regeleinheit dazu eingerichtet ist, die Temperatur und/oder die Luftfeuchtigkeit des jeweiligen Luftstroms basierend auf dem jeweiligen Wert des Sensors zu steuern/regeln.

Entsprechend kann nicht nur beim Abweichen von einem Soll-Wert der Volumenstrom geregelt, sondern können die Lufteigenschaften direkt an die erwünschten Eigenschaften beziehungsweise Bedingungen angepasst werden. Beispielsweise kann die durchgeführte Klimatisierung von der jeweiligen Wetterlage und der Jahreszeit abhängen, insbesondere wenn beispielsweise Außenluft für die Zuluft verwendet wird. So kann im Sommer beispielsweise eine Nachkühlung für die Klimatisierung der jeweiligen Behandlungsvorrichtung erforderlich sein während im Winter eine Befeuchtung der Luft eine verbesserte Atmungsluft für den jeweiligen Patienten bereitstellt.

Alternativ oder zusätzlich zu der sensorbasierten Regelung des jeweiligen Luftstroms, kann die Medienversorgungseinrichtung ebenfalls ein Bedienelement aufweisen, welches mit der Steuer-/Regeleinheit kommunikativ verbunden und zum Einstellen eines Soll-Werts der Temperatur, des jeweiligen Volumenstroms, der CO₂-Konzentration, und/oder der relativen Luftfeuchtigkeit eingerichtet ist.

Beispielsweise kann das Bedienelement als Fernbedienung ausgebildet sein, welche mit der Steuer-/Regeleinheit verbunden ist, sodass ein Patient oder ein Benutzer den jeweiligen Soll-Wert manuell und abhängig von den erwünschten Eigenschaften einstellen kann.

Bevorzugt ist zum Einstellen der Temperatur, des Flusses und/oder der Feuchtigkeit ein drahtloses Bedienelement vorgesehen, welches mit der Steuer-/Regeleinheit kommunikativ verbunden ist, bevorzugt über Funk, WLAN, Bluetooth, Zigbee, NFC, Wi-Fi, RFID oder Infrarot, wobei das Bedienelement bevorzugt als Endgerät, bevorzugt als Smartphone, Smartwatch oder Tablet, ausgebildet ist, und wobei die Steuer-/Regeleinheit bevorzugt eingerichtet ist, den Soll-Wert basierend auf einer Kennung des Benutzers einzustellen.

Beispielsweise kann ein Patient somit einfach über ein vorhandenes Endgerät wie ein Smartwatch oder Tablet eine Einstellung der Klimatisierungsvorrichtung und insbesondere des Volumenstroms des jeweiligen Ventilators vornehmen, wobei die Steuer-/Regeleinheit den Volumenstrom basierend auf der vorgenommenen Einstellung und eines Status des jeweiligen Ventilators, beispielsweise anhand eines gemessenen Durchflusses, mittels eines Regelwerts beziehungsweise Stellwerts entsprechend regelt.

Zum Einstellen des Soll-Werts ist weiterhin bevorzugt eine Kopplung des Endgeräts mit der Steuer-/Regelung erforderlich, welche über eine entsprechende Anmeldung erfolgen kann. Eine solche Anmeldung kann über eine manuelle Benutzereingabe oder auch automatisch erfolgen, beispielsweise über das automatische Senden einer Identifikation, beispielsweise einer Patientenidentifikation oder des medizinischen Personals, bevorzugt über NFC oder via RFID. Sowohl die Benutzerkennung als auch die jeweiligen Einstellungen können weiterhin in dem Endgerät und/oder in der Steuer-/Regeleinheit gespeichert sein, sodass eine wiederholte Eingabe bei zukünftiger Verwendung in einem Dialysesystem nicht erforderlich ist.

Die Medienversorgungseinrichtung bietet somit eine Vielzahl an Möglichkeiten, sowohl eine erwünschte, verbesserte Klimatisierung als auch die technische Schnittstelle für ein medizinisches Behandlungsgerät je nach Bedarf bereitzustellen.

Bevorzugt kann in einer Weiterbildung oder Alternative die Klimatisierungsvorrichtung an der Medienversorgungseinrichtung einen Klimatisierungsanschluss bereitstellen, an welchen das zu klimatisierende medizinische Behandlungsgerät anschließbar ist. Damit kann beispielsweise über ein Kühlmedium Kälte direkt in das medizinische Behandlungsgerät eingespeist werden und auf diese Weise eine direkte Kühlung erreicht werden. Das medizinische Behandlungsgerät kann auf diese Weise entsprechend an einen an der Medienversorgungseinrichtung bereitgehaltenen Kühlkreislauf angeschlossen werden und auf diese Weise gekühlt werden. Das am Klimatisierungsanschluss bereitgestellte Kühlmedium kann entweder Luft sein, oder ein anderes Kühlmedium - beispielsweise Kühlwasser.

Die oben gestellte Aufgabe wird weiterhin durch ein medizinisches Behandlungssystem mit mindestens einem medizinischen Behandlungsgerät, bevorzugt mit einer Hämodialysevorrichtung, einer Hämofiltrationsvorrichtung, einer Hämodiafiltrationsvorrichtung oder einer Apheresevorrichtung, gemäß Anspruch 15 gelöst. Erfindungsgemäß ist mindestens eine Medienversorgungseinrichtung mit einer Klimatisierungsvorrichtung wie bereits oben beschrieben vorgesehen.

### Kurze Beschreibung der Figuren

Bevorzugte weitere Ausführungsformen der Erfindung werden durch die nachfolgende Beschreibung der Figuren näher erläutert. Dabei zeigen:
- Figur 1: eine schematische Darstellung der Wirkung einer Klimatisierungsvorrichtung mit einem Abluftkanal;
- Figur 2: eine schematische Darstellung einer Klimatisierung einer extrakorporalen Blutbehandlungsvorrichtung mit einem Zuluftkanal und einer Steuer-/Regeleinheit;
- Figur 3: eine Ausgestaltung einer Medienversorgungseinrichtung mit einer Klimatisierungsvorrichtung;
- Figur 4: eine schematische Teilansicht der Ausführungsform gemäß Figur 3 mit alternativen Anschlüssen und Sensoren; und
- Figur 5: eine schematische Darstellung einer Medienversorgungseinrichtung für zwei extrakorporale Blutbehandlungsvorrichtungen.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente in den unterschiedlichen Figuren mit identischen Bezugszeichen versehen, und auf eine wiederholte Beschreibung dieser Elemente wird teilweise verzichtet, um Redundanzen zu vermeiden.

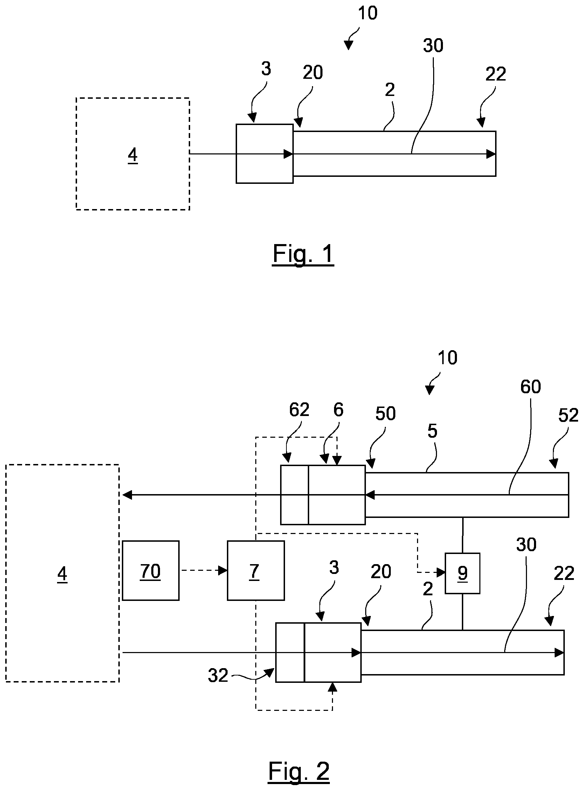

In Figur 1 ist schematisch eine Klimatisierungsvorrichtung 10 dargestellt, welche eine zonenbezogene Klimatisierung für einen Behandlungsraum bereitstellt, in welchem ein medizinisches Behandlungsgerät angeordnet ist. Die Klimatisierungsvorrichtung 10 ist in einer in dieser Figur nicht vollständig gezeigten Medienversorgungseinrichtung zur Versorgung des medizinischen Behandlungsgeräts mit mindestens einem Medium angeordnet. Das medizinische Behandlungsgerät kann beispielsweise eine Dialysevorrichtung sein, welche mit der Medienversorgungseinrichtung zur Versorgung der Dialysevorrichtung mit beispielsweise Dialysewasser, Konzentrat und/oder Strom über geeignete Schnittstellen verbunden ist. Auf die Medienversorgungseinrichtung wird weiter unten eingegangen werden.

Das medizinische Behandlungsgerät 4 ist schematisch durch den gestrichelten Bereich in der Figur angedeutet.

Die Klimatisierungsvorrichtung 10 umfasst in dem vorliegend gezeigten Ausführungsbeispiel einen Abluftkanal 2 sowie einen daran angebrachten ersten Ventilator 3. Der Ventilator 3 ist an einem ersten Ende 20 des Abluftkanals 2 angeordnet und ist derart eingerichtet, dass dieser Luft in den Abluftkanal 2 von dem ersten Ende 20 zu einem zweiten Ende 22 fördert, sodass entsprechend ein Luftstrom 30 bereitgestellt wird, wie durch den Pfeil angedeutet.

Der Luftstrom 30 umfasst somit Luft, welche von dem ersten Ventilator 3 in den Abluftkanal 2 angesaugt wird und fördert damit Umgebungsluft aus der Umgebung des ersten Ventilators 3 beziehungsweise aus der Umgebung des ersten Endes 20 des Abluftkanals 2, wie ebenfalls durch den Pfeil angedeutet.

Durch die Anordnung und Ausrichtung des Ventilators 3 und des ersten Endes 20 des Abluftkanals 2 kann somit gezielt Umgebungsluft aus einem bestimmten Bereich eines Behandlungsraums aufgenommen und über den Abluftkanal 2 aus dem Behandlungsraum abgeführt werden. Das zweite Ende 22 ist dazu bevorzugt mit der Außenluft verbunden und kann entsprechend mit einem zentralen Sammelkanal verbunden werden.

Alternativ oder zusätzlich kann das zweite Ende 22 mittels eines Verbindungskanals an andere Luftkanäle gekoppelt werden, sodass die vom Luftstrom 30 abgeführte Abluft insgesamt eine größere Distanz als der Abluftkanal 2 selbst überbrücken kann.

Dadurch, dass der Luftstrom 30 Umgebungsluft aufnimmt und dies durch die Ausrichtung und Anordnung des Ventilators 3 beziehungsweise des Abluftkanals 2 selektiv erfolgt, kann Luft von einem zu dem ersten Ventilator 3 beziehungsweise des ersten Endes 20 des Abluftkanals benachbarten medizinischen Behandlungsgerät 4 abgeführt werden.

Das medizinische Behandlungsgerät 4 ist nicht Teil der Klimatisierungsvorrichtung 10, wie mit den gestrichelten Linien angedeutet, kann jedoch optional Teil eines entsprechenden Behandlungssystems sein. Je nach Betriebsmodus des medizinischen Behandlungssystems 4 kann Wärme erzeugt werden, welche zu einer lokalen Erwärmung der Umgebungsluft führt. Beispielsweise muss bei einem medizinischen Behandlungsgerät in Form einer Dialysevorrichtung das Dialysemedium erwärmt werden, wozu eine entsprechende Heizvorrichtung in der Dialysevorrichtung vorhanden sein muss. Die bei dem Vorgang des Erwärmens des Dialysemediums abfallende Wärme kann durch die vorgeschlagene Klimatisierungsvorrichtung 10 abgeführt werden. Beispielsweise kann durch den Ventilator 3 und den Abluftkanal 2 diese Umgebungsluft beziehungsweise diese Wärme selektiv abgeführt, sodass entsprechend eine lokale, dezentrale Klimatisierung für das medizinische Behandlungsgerät 4 bereitgestellt wird.

Der Ventilator 3 kann dabei kontinuierlich angeschaltet sein und dabei einen ebenfalls kontinuierlichen Volumenstrom für den Luftstrom 30 bereitstellen. Es kann optional jedoch auch vorgesehen sein, dass der Ventilator 3 nur nach einer vorgegebenen Betriebszeit eingeschaltet wird, um somit beispielsweise Energie zu sparen oder Behandlungszeiten zu berücksichtigen.

In Figur 2 ist eine schematische Darstellung der Klimatisierungsvorrichtung 10 gemäß Figur 1 gezeigt, wobei die Klimatisierungsvorrichtung 10 in einer nicht detailliert gezeigten Medienversorgungseinrichtung angeordnet ist und wobei zusätzlich ein Zuluftkanal 5 vorgesehen ist. Wie für den Abluftkanal 2 ist ein zweiter Ventilator 6 für den Zuluftkanal 5 vorgesehen, welcher an einem entsprechenden ersten Ende 50 des Zuluftkanals 5 angeordnet ist.

Der zweite Ventilator 6 wirkt jedoch genau in die entgegengesetzte Richtung, sodass Zuluft von einem entsprechenden zweiten Ende 52 des Zuluftkanals 5 zu dessen erstem Ende 50 gefördert und ein entsprechender Luftstrom 60 bereitstellt wird, wie mit dem Pfeil angedeutet. Entsprechend wird Zuluft in den Raum eingebracht, welche sich mit der Umgebungsluft vermischt, wie ebenfalls mit dem Pfeil angedeutet.

Wie in Figur 1 gezeigt, ist auch in der Ausführungsform gemäß Figur 2 ein medizinisches Behandlungsgerät 4 vorgesehen, welches bezüglich der Klimatisierungsvorrichtung 10 und damit auch bezüglich der Medienversorgungseinrichtung derart positioniert ist, dass es mit dem jeweiligen ersten Ende 20, 50 des Abluftkanals 2 und des Zuluftkanals 5 ausgerichtet ist. Es sind jedoch auch alternative Anordnungen denkbar, wobei das medizinische Behandlungsgerät 4 beispielsweise näher zum ersten Ende 50 des Zuluftkanals 5 hin ausgerichtet ist, um eine Prallkühlung bereitzustellen und wobei optional der erste Ventilator 3 eine entsprechende höhere Drehzahl aufweisen kann, um eine hinreichende Ansaugwirkung bereitzustellen.

An jedem Ventilator kann weiterhin ein Schalldämpfer 32, 62 angebracht sein, sodass die zusätzliche Belüftung beziehungsweise Entlüftung keine unangenehme Geräusche für den zu behandelnden Patienten verursacht.

Weiterhin ist eine Steuer-/Regeleinheit 7 vorgesehen, welche mit den Ventilatoren 3, 6 verbunden ist und einen jeweiligen Volumenstrom durch den jeweiligen Luftkanal durch entsprechende Einstellung der Drehzahl regelt, wie mit den gestrichelten Pfeilen gezeigt. Die Steuer-/Regeleinheit 7 ist mit einem Temperatursensor 70 kommunikativ verbunden, wobei der Temperatursensor 70 an dem medizinischen Behandlungsgerät 4 angebracht ist.

Der Temperatursensor 70 ist dabei dazu eingerichtet, die entsprechenden Messwerte drahtlos an die Steuer-/Regeleinheit 7 zu übermitteln, wie mit dem gestrichelten Pfeil gezeigt, vorliegend über Bluetooth. Es können jedoch ebenfalls andere drahtlose Kommunikationsverbindungen gewählt werden, wie vorstehend beschrieben. Der Temperatursensor 70 kann entweder batteriebetrieben sein oder über eine entsprechende elektrisch leitfähige Verbindung an eine Stromversorgung des medizinischen Behandlungsgeräts 4 angeschlossen sein.

In einer in den Figuren nicht explizit gezeigten Alternative kann der Temperatursensor 70 auch über eine Kabelverbindung mit der Steuer-/Regeleinheit 7 verbunden sein. Dazu kann der Temperatursensor 70 beispielsweise in Form eines Wurfthermometers ausgebildet sein, welches beispielsweise einen in einem Sensorgehäuse angeordneten Temperatursensor 70 aufweist, der dann über ein flexibles Kabel an die Steuer-/Regeleinheit 7 angebunden sein kann. Ein solches Wurfthermometer kann dann über einen Teil des medizinischen Behandlungsgeräts 4 gehängt werden, so dass es dann die im Bereich des eigentlichen Temperatursensors 70 gewonnenen Temperaturwerte über die Kabelverbindung an die Steuer-/Regeleinheit 7 übergibt.

Bei Verwendung eines kabelbehafteten Temperatursensors 70 kann an der Medienversorgungseinrichtung ein entsprechender Anschluss, beispielsweise in Form einer Steckerbuchse, vorgesehen sein, an welchen ein dazu korrespondierender Anschluss des Temperatursensors 70 beziehungsweise dessen Kabel angeschlossen werden kann, beispielsweise durch einen in die Steckerbuchse einsteckbaren Stecker. Die Weiterleitung der Temperaturwerte an die Steuer-/Regeleinheit 7 findet dann entsprechend durch die Medienversorgungseinrichtung statt.

Durch die Verwendung eines kabelgebundenen Temperatursensors 70 kann auf die Verwendung von Energiespeichern, beispielsweise Batterien, in dem Temperatursensor 70 zur Energieversorgung einer drahtlosen Kommunikationsverbindung verzichtet werden, so dass sich auf diese Weise eine strahlungsarme, zuverlässige und kostengünstige Temperaturbestimmung an dem medizinischen Behandlungsgerät 4 erreichen lässt. Das zusätzlich verwendete Kabel ist dabei nicht notwendiger Weise hinderlich, da das medizinische Behandlungsgerät 4 ohnehin über Kabel und Leitungen an der Medienversorgungseinrichtung angeschlossen ist und das Vorliegen eines weiteren Kabels daher nicht ins Gewicht fällt.

Die Steuer-/Regeleinheit 7 empfängt somit die gemessenen Temperaturwerte des medizinischen Behandlungsgeräts 4. In der Steuer-/Regeleinheit 7 sind entsprechende Soll-Werte hinterlegt, welche mit den empfangenen Ist-Werten verglichen werden und wobei die Steuer-/Regeleinheit 7 eine entsprechende Stellgröße beziehungsweise Regelgröße ausgibt, um die Drehzahl des jeweiligen Ventilators 3, 6 entsprechend zu ändern.

Folglich wird durch die Steuer-/Regeleinheit 7 und den Temperatursensor 70 eine genauere Klimatisierung ermöglicht, wobei die Zuluft und die Abluft entsprechend des eingestellten Volumenstroms eine lokale Kühlung des medizinischen Behandlungsgeräts 4 bewirken.

Zusätzlich ist eine Drosselkappe 9 zwischen dem Abluftkanal 2 und dem Zuluftkanal 5 vorgesehen, welche ebenfalls von der Steuer-/Regeleinheit 7 geregelt wird, wie mit dem gestrichelten Pfeil gezeigt. Die Drosselkappe ist im Normalbetrieb geschlossen, kann jedoch entweder in Zwischenperioden geöffnet sein, um beispielsweise Abluft mit Zuluft zu mischen und somit eine Nachkühlung vor der Abgabe an beispielsweise Außenluft oder benachbarte Systeme bereitzustellen. Ebenfalls kann die Drosselklappe 9 die Zuluft vorwärmen, beispielsweise wenn im Winter kalte Außenluft eingespeist und diese entweder als zu kalt empfunden oder für die jeweiligen Komponenten beziehungsweise für die medizinische Behandlung schädlich sein könnte, beispielsweise weil zu kalte Außenluft zu vermehrter Kondensation von Luftfeuchtigkeit an unerwünschten Orten oder in noch extremeren Fällen zum Festfrieren beweglicher Teile führen könnte.

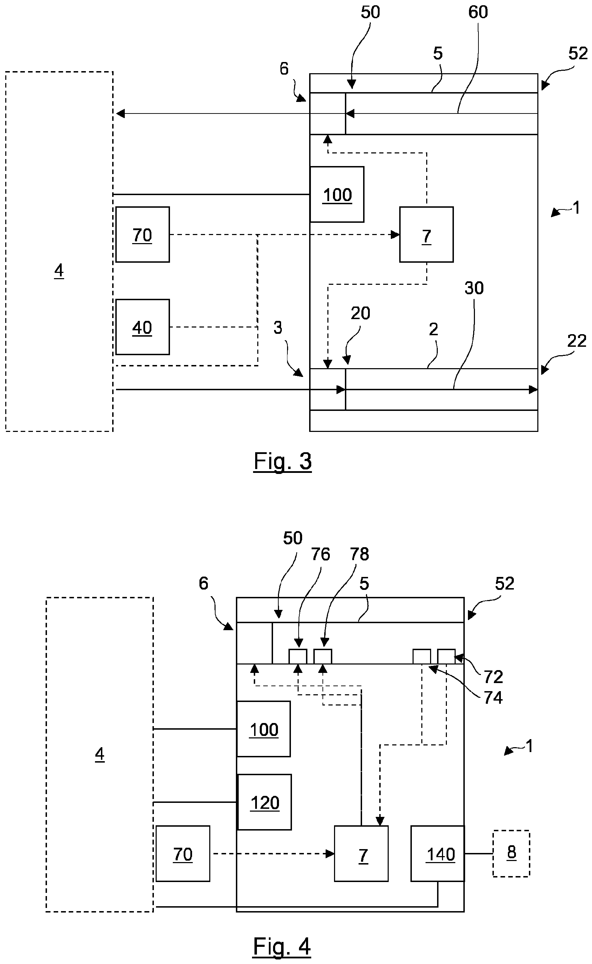

Die Ausführungsform gemäß Figur 3 entspricht im Wesentlichen der Ausführungsform gemäß Figur 2, wobei jedoch eine Drosselklappe nicht explizit vorgesehen, jedoch optional möglich ist. Sowohl der Zuluftkanal 5 als auch der Abluftkanal 2 sowie die entsprechenden Ventilatoren 3, 6 und die Steuer-/Regeleinheit 7 sind gemäß Figur 3 in einer Medienversorgungseinrichtung 1 angeordnet.

Die Medienversorgungseinrichtung 1 dient zur Versorgung eines medizinischen Behandlungsgeräts 4 mit den entsprechenden Betriebsmedien. Das medizinische Behandlungsgerät 4 ist dabei über entsprechende, beispielsweise medienspezifische, Schnittstellen an die Medienversorgungseinrichtung 1 angeschlossen.

Über die Medienversorgungseinrichtung 1 kann beispielsweise ein als Dialysevorrichtung ausgebildetes medizinisches Behandlungsgerät 4 mit Dialysewasser, Dialysekonzentrat und Strom versorgt werden. Weiterhin kann auch eine Ableitung für verbrauchte Dialyseflüssigkeit vorgesehen sein. Eine Kopplung des medizinischen Behandlungsgeräts an die Medienversorgungseinrichtung 1 findet dabei beispielsweise über Schläuche und Kabel an entsprechenden Schnittstellen der Medienversorgungseinrichtung 1 statt, welche vorliegend über Anschlüsse 100 an der Medienversorgungseinrichtung 1 angekoppelt sind.

Die Medienversorgungseinrichtung 1 kann mit einem eigenen Abdeckungspaneel ausgebildet sein, so dass sie in eine entsprechende Ausnehmung in einer Wand oder in einer Wandvorsatzschale eingebaut werden kann. Das Abdeckungspaneel überdeckt dabei die Ausnehmung in der Wand oder Wandvorsatzschale, so dass mittels des Abdeckungspaneels zum einen eine geschlossene Wandkontur ausgebildet werden kann, was aus hygienischen Gründen wünschenswert ist und die einzelnen Anschlüsse und Auslässe der Medienversorgungseinrichtung 1 befinden sich in einer vorgegebenen und bevorzugten Anordnung zueinander. Zum anderen kann die Ausnehmung in der Wand oder Wandvorsatzschale so ästhetisch ansprechend verschlossen werden.

Die Medienversorgungseinrichtung 1 kann auch mit einem eigenen Gehäuse ausgebildet sein, in welchem sich Komponenten der Medienversorgungseinrichtung 1 befinden und welches auf einer Wandfläche befestigt werden kann. Damit kann eine Montage der Medienversorgungseinrichtung auch auf Wandabschnitten vorgenommen werden, welche nur eine geringe Einbautiefe ermöglichen.

Die Medienversorgungseinrichtung 1 kann in einer weiteren Alternative auch ohne ein eigenes Abdeckungspaneel bereitgestellt werden, so dass die einzelnen Anschlüsse und Auslässe dann in einer vorhandenen oder bereitzustellenden Abdeckung aufgenommen werden können. Auf diese Weise kann eine bereits vorhandene Anmutung eines Wandbereichs besser aufrechterhalten bleiben beziehungsweise die Anschlüsse und Auslässe der Medienversorgungseinrichtung 1 können in eine bereits vorhandene Infrastruktur einfacher integriert werden.

Durch den Anschluss 100 kann das medizinische Behandlungsgerät 4 entsprechend mit einem Fluid versorgt werden, insbesondere mit einer medizinischen Flüssigkeit. Das medizinische Behandlungsgerät 4 ist vorliegend als Dialysegerät ausgebildet, wobei das durch den Anschluss 100 der Medienversorgungseinrichtung 1 bereitgestellte Fluid eine Dialysierflüssigkeit sein kann. Es können jedoch ebenfalls andere Flüssigkeiten, insbesondere medizinische Flüssigkeiten, Konzentrate und/oder Wasser bereitgestellt werden. Obwohl nicht in Figur 3 gezeigt, können ebenfalls mehrere Anschlüsse 100 für die jeweiligen Fluide vorgesehen sein.

Weiterhin kann in der Medienversorgungseinrichtung 1 - wie bereits erwähnt - auch eine Energiezufuhr beispielsweise mittels eines Stromanschlusses stattfinden.

Die Ventilatoren 3, 6 sind gemäß Figur 3 in dem jeweiligen Luftkanal angeordnet, sodass die Klimatisierungsvorrichtung 10 und insbesondere die Medienversorgungseinrichtung 1 kompakter ausgebildet werden können. Die Anordnung innerhalb des jeweiligen Luftkanals hat weiterhin den Vorteil, dass der Wirkungsgrad erhöht und gleichzeitig Geräusche aufgrund der Anordnung und der Schalldämmung durch die Medienversorgungseinrichtung 1 bereits minimiert werden können. Es können jedoch zusätzlich Schalldämpfer vorgesehen sein (nicht gezeigt).

Die Steuer-/Regeleinheit 7 ist weiterhin kommunikativ mit dem medizinischen Behandlungsgerät verbunden, wie mit dem entsprechenden, gestrichelten Pfeil gezeigt. Entsprechend ist die Steuer-/Regeleinheit 7 dazu eingerichtet, ein Betriebsparameter von dem medizinischen Behandlungsgerät zu empfangen. Dies hat unter anderem den Vorteil, dass die Steuer-/Regeleinheit 7 Informationen bezüglich eines Betriebsmodus erhält, sodass die Regelung der Ventilatoren 3, 6 nicht nur anhand der gemessenen Temperatur, sondern ebenfalls in Abhängigkeit von dem bestimmten Betriebsmodus erfolgt.

Beispielsweise kann am Ende der Behandlung eine Desinfektion des medizinischen Behandlungsgeräts mittels einer Heiß-Desinfektion vorgesehen sein, welche die Umgebungstemperatur erheblich steigen lässt. Durch die entsprechende Kommunikation mit der Steuer-/Regeleinheit 7 wird jedoch sichergestellt, dass der Volumenstrom der Abluft und der Zuluft bereits erhöht wird, sodass eine plötzliche Erwärmung antizipiert werden kann und der Patient entsprechend keine unangenehme Temperatursteigung der Umgebungsluft empfindet.

Weiterhin ist auch ein Bedienelement 40 in Form eines Smartphones vorgesehen, über das der Patient oder ein sonstiger Benutzer eine Einstellung der gewünschten Temperatur beziehungsweise des Volumenstroms eingeben kann. Das Smartphone ist entsprechend drahtlos mit der Steuer-/Regeleinheit 7 verbunden und zwar bevorzugt über NFC oder RFID, sodass eine Kennung beziehungsweise eine Identifikation des Benutzers automatisch und eine entsprechende Anmeldung des Benutzers ebenfalls automatisch erfolgt. Anhand der Benutzereinstellung und der gemessenen Temperatur wird von der Steuer-/Regeleinheit 7 eine entsprechende Stellgröße eingegeben und der Volumenstrom im jeweiligen Luftkanal entsprechend geregelt.

In Figur 4 ist schematisch eine Teilansicht der Ausführungsform gemäß Figur 3 gezeigt, wobei die entsprechenden Merkmale des Abluftkanals sowie die Details des Zuluftkanals 5 lediglich für eine verbesserte Übersicht weggelassen wurden.

In dem Zuluftkanal 5 sind weitere Sensoren angeordnet, welche Eigenschaften der eingespeisten Zuluft messen. Vorliegend sind ein weiterer Temperatursensor 72 sowie ein Luftfeuchtigkeitssensor 74 vorgesehen, welche entsprechende Messwerte an die Steuer-/Regeleinheit 7 weiterleiten, wie mit den gestrichelten Pfeilen gezeigt. Um die entsprechenden Eigenschaften der Zuluft zu regeln, sind weiterhin ein Kühlelement 76 sowie ein Befeuchtungselement 78 in dem Zuluftkanal 5 vorgesehen, welche von der Steuer-/Regeleinheit 7 anhand der jeweiligen gemessenen Werte angesprochen werden. Die Steuer-/Regeleinheit 7 gibt entsprechend eine Regelgröße beziehungsweise Stellgröße aus, welche auf einem Vergleich des empfangenen Ist-Werts mit einem hinterlegten Soll-Wert basiert.

Somit kann beispielsweise warme Außenluft, beispielsweise im Sommer, entsprechend vorgekühlt werden, sodass eine hinreichende Kühlung beziehungsweise Klimatisierung der angekoppelten extrakorporalen Blutbehandlungsvorrichtung 4 erfolgen kann. Ebenfalls kann eine Befeuchtung gerade im Winter für trockene Außenluft vorgesehen sein, sodass für den Patienten gleichzeitig eine angenehme Atmungsluft bereitgestellt wird. Es können jedoch auch weitere Sensoren vorgesehen sein, beispielsweise Durchflussmesser oder CO₂-Sensoren, und die Anordnung ist nicht auf den Zuluftkanal 5 beschränkt, sondern kann ebenfalls im Abluftkanal vorgesehen sein.

Zusätzlich zum Fluidanschluss 100 ist weiterhin ein Ablaufanschluss 120 vorgesehen, womit Fluide aus dem medizinischen Behandlungsgerät 4 abgeführt und entsorgt werden können. Beispielsweise können somit Fluide wie verbrauchtes Dialysat oder Retentat oder auch Kalibrierflüssigkeiten auf einfache Weise entsorgt werden, ohne dass es weitere Anpassungen der Anordnung des medizinischen Behandlungsgeräts 4 bezüglich der Medienversorgungseinrichtung 1 bedarf. Somit wird sichergestellt, dass eine vorgesehene, zonenbezogene Klimatisierung erfolgen kann.

Weiterhin ist in der Medienversorgungseinrichtung 1 ein Elektromodul 140 vorgesehen, welches das medizinische Behandlungsgerät 4 mit einer Stromquelle 8 verbindet. Dies hat den Vorteil, dass eine Stromversorgung zu jedem Zeitpunkt garantiert werden kann und ein Benutzer nicht von längeren Stromkabeln oder dem Vorhandsein von entsprechenden Fassungen abhängig ist. Die Medienversorgungseinrichtung 1 ist somit quasi autark und bietet die Sicherheit, dass ein neuplatziertes medizinisches Behandlungsgerät 4 mit den für die Behandlung gewünschten Medien versorgt werden kann.

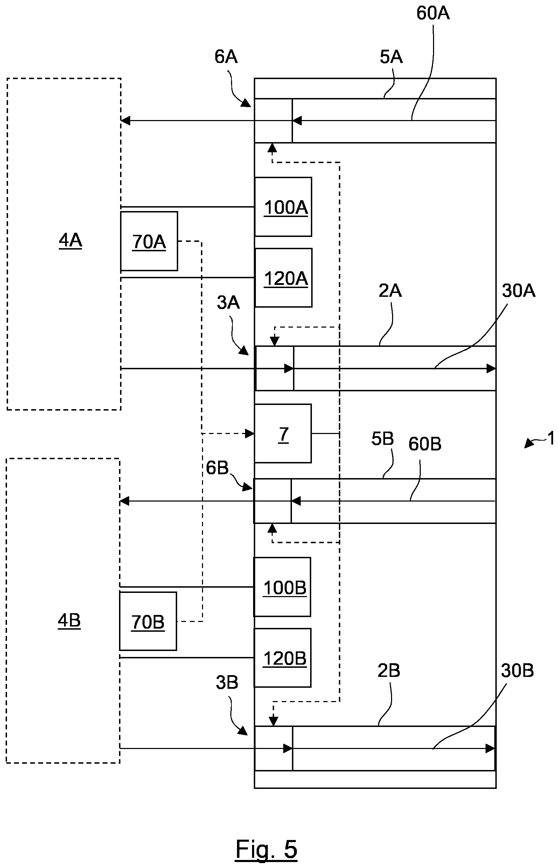

In Figur 5 ist schematisch eine Medienversorgungseinrichtung 1 für zwei medizinische Behandlungsgeräte 4A, 4B gezeigt, welche vorliegend als Dialysegeräte ausgebildet sind.

Abgesehen von den jeweiligen Temperatursensoren 70A, 70B sind dabei alle Komponenten der Medienversorgungseinrichtung 1 und insbesondere der Klimatisierungsvorrichtung in der Medienversorgungseinrichtung 1 angeordnet.

Entsprechend ist für jedes angekoppelte Dialysegerät ein Zuluftkanal 5A, 5B mit entsprechendem Ventilator 6A, 6B zum Bereitstellen eines jeweiligen Luftstroms 60A, 60B vorgesehen. Auf analoge Weise umfasst Medienversorgungseinrichtung 1 ebenfalls ein Abluftkanal 2A, 2B mit entsprechendem Ventilator 3A, 3B zum Bereitstellen eines jeweiligen Luftstroms 30A, 30B. Die Ventilatoren 3A, 3B, 6A, 6B sind dabei kommunikativ mit der Steuer-/Regeleinheit 7 verbunden, sodass die Steuer-/Regeleinheit 7 den entsprechenden Volumenstrom anhand des empfangenen Messwerts von dem jeweiligen Temperatursensor 70A, 70B einstellen beziehungsweise regeln kann.

Die Ausgestaltung für zwei Dialysegeräte hat somit den Vorteil, dass das Dialysesystem lediglich eine Steuer-/Regeleinheit 7 bedarf und die Medienversorgungseinrichtung 1 somit kompakter und ohne Redundanz ausgebildet werden kann.

Die Zuluftkanäle 5A, 5B und Abluftkanäle 2A, 2B sind weiterhin derart angeordnet, dass eine Klimatisierung des angeschlossenen Dialysegeräts bereitgestellt werden kann. Um dies zu vereinfachen und das entsprechende Dialysegerät entsprechend anzuordnen beziehungsweise auszurichten können weiterhin Markierungen vorgesehen sein (nicht gezeigt), beispielsweise auf dem Boden eines Dialyseraums, welche die korrekte Positionierung anzeigen und somit die Vorbereitung der Behandlung und ggf. einer Korrektur der Klimatisierung unterstützen.

Weiterhin sind für jedes Dialysegerät ein Fluidanschluss 100A, 100B sowie ein Ablaufanschluss 120A, 120B vorgesehen, sodass das jeweilige Dialysegerät mit einem erforderlichen Fluid versorgt und ein verwendetes beziehungsweise verbrauchtes Fluid entsprechend abgeführt beziehungsweise entsorgt werden kann. Entsprechend können die jeweiligen Anschlüsse mit einem zentralen Fluidsystem verbunden sein oder es kann ein Reservoir mit einem entsprechenden Füllstandindikator vorgesehen sein, welche das vorhandene Fluid anzeigt.

Die Ausgestaltung für zwei medizinische Behandlungsgeräte 4A, 4B hat weiterhin den Vorteil, dass die Medienversorgungseinrichtung 1 bereits bei einem angeschlossenen medizinischen Behandlungsgerät eine erforderliche, individuelle und lokale Klimatisierung bereitstellen kann, es aber zusätzlich, je nach Bedarf, ein weiteres medizinisches Behandlungsgerät zugeschaltet werden kann, um beispielsweise einen weiteren Patienten zu behandeln. Auch in diesem Fall wird eine individuelle und lokale Klimatisierung bereitgestellt, sodass die Wärmeerzeugung des einen medizinischen Behandlungsgeräts die Umgebungsluft des anderen medizinischen Behandlungsgeräts nicht oder nur unwesentlich beeinflusst. Entsprechend können lokale Änderungen der Umgebungsluft ebenfalls lokal entgegengewirkt werden, ohne dass es dabei eine zentrale Klimatisierung bedarf.

Die durch die Klimatisierungsvorrichtung bereitgestellte Klimatisierung ist in den vorstehend beschriebenen Ausführungsformen in Bezug auf ein medizinisches Behandlungsgerät gezeigt, wobei das medizinische Behandlungsgerät nicht Teil der Medienversorgungseinrichtung 1 ist.

Es kann jedoch optional vorgesehen sein, dass die Medienversorgungseinrichtung 1 als Zusatzmodul für ein medizinisches Behandlungsgerät 4, beispielsweise für eine extrakorporale Blutbehandlungsvorrichtung, ausgebildet ist, beispielsweise als separate, aber schaltbare und ankoppelbare Einrichtung, sodass diese ein gemeinsames System ausbilden. Entsprechend kann beispielsweise optional ein Dialysesystem vorgesehen sein, welches sowohl eine extrakorporale Blutbehandlungsvorrichtung als auch die vorstehend beschriebene Medienversorgungseinrichtung umfasst.

Soweit anwendbar, können alle einzelnen Merkmale, die in den Ausführungsbeispielen dargestellt sind, miteinander kombiniert und/oder ausgetauscht werden, ohne den Bereich der Erfindung zu verlassen.

### Bezugszeichenliste

- 1: Medienversorgungseinrichtung
- 10: Klimatisierungsvorrichtung
- 100: Fluidversorgungsanschluss
- 100A , 100B: Fluidversorgungsanschluss
- 120: Ablaufanschluss
- 120A, 120B: Ablaufanschluss
- 140: Elektromodul
- 2: Abluftkanal
- 2A, 2B: Abluftkanal
- 20: erstes Ende
- 22: zweites Ende
- 3: erster Ventilator
- 3A, 3B: erster Ventilator
- 30: Luftstrom
- 30A, 30B: Luftstrom
- 32: Schalldämpfer
- 4: medizinisches Behandlungsgerät
- 4A, 4B: medizinisches Behandlungsgerät
- 40: Bedienelement
- 5: Zuluftkanal
- 5A, 5B: Zuluftkanal
- 50: erstes Ende
- 52: zweites Ende
- 6: zweiter Ventilator
- 6A, 6B: zweiter Ventilator
- 60: Luftstrom
- 60A, 60B: Luftstrom
- 62: Schalldämpfer
- 7: Steuer-/Regeleinheit
- 70: Temperatursensor
- 70A, 70B: Temperatursensor
- 72: Temperatursensor
- 74: Feuchtigkeitssensor
- 76: Kühlelement
- 78: Befeuchtungselement
- 8: Stromversorgung
- 9: Drosselklappe

## Patentansprüche

1. Medienversorgungseinrichtung (1) zur Versorgung eines medizinischen Behandlungsgeräts (4) mit einem Medium,
**gekennzeichnet durch**
eine Klimatisierungsvorrichtung (10) zur Klimatisierung eines an die Medienversorgungseinrichtung (1) angeschlossenen und separaten medizinischen Behandlungsgeräts (4), wobei die Klimatisierungsvorrichtung (10) einen Abluftkanal (2; 2A, 2B) zum Abführen von Luft aus der Umgebung des medizinischen Behandlungsgeräts (4) umfasst, wobei ein erster Ventilator (3; 3A, 3B) zum Abführen von Luft aus der Umgebung des medizinischen Behandlungsgeräts (4) in den Abluftkanal (2; 2A, 2B) hinein vorgesehen ist.

2. Medienversorgungseinrichtung (1) gemäß Anspruch 1, **gekennzeichnet durch** einen Anschluss (100) zum Anschließen des medizinischen Behandlungsgeräts (4) zum Zuführen eines Mediums zu dem medizinischen Behandlungsgerät (4) und/oder einen Ablaufanschluss (120) zum Abführen eines Mediums von dem medizinischen Behandlungsgerät (4).

3. Medienversorgungseinrichtung (1) gemäß einem der vorstehenden Ansprüche, **dadurch gekennzeichnet, dass** die Klimatisierungsvorrichtung (10) einen Temperatursensor (70; 70A, 70B) zum Messen der Temperatur des medizinischen Behandlungsgeräts (4; 4A, 4B) und/oder der Umgebungstemperatur des medizinischen Behandlungsgeräts (4; 4A, 4B) aufweist und eine mit dem Temperatursensor (70; 70A, 70B) kommunikativ verbundene Steuer-/Regeleinheit (7) dazu eingerichtet ist, die Klimatisierungsleistung der Klimatisierungsvorrichtung (10) basierend auf einem Wert des Temperatursensors (70; 70A, 70B) zu steuern/regeln.

4. Medienversorgungseinrichtung (1) gemäß Anspruch 3, **dadurch gekennzeichnet, dass** die Steuer-/Regeleinheit (7) dazu eingerichtet ist, einen Betriebsparameter oder einen mit einem bestimmten Betriebsmodus übereinstimmenden Betriebsparameter des medizinischen Behandlungsgeräts (4) zu empfangen und die Klimatisierungsleistung basierend auf dem Betriebsparameter zu steuern/regeln.

5. Medienversorgungseinrichtung (1) gemäß Anspruch 3 oder 4, **dadurch gekennzeichnet, dass** die Steuer-/Regeleinheit (7) dazu eingerichtet ist, die Klimatisierungsleistung über einen Volumenstrom eines jeweiligen mit der Steuer-/Regeleinheit (7) verbundenen Ventilators zu steuern/regeln.

6. Medienversorgungseinrichtung (1) gemäß einem der vorstehenden Ansprüche, **dadurch gekennzeichnet, dass** die Klimatisierungsvorrichtung (10) einen Zuluftkanal (5; 5A, 5B) zum Zuführen von Luft in die Umgebung des medizinischen Behandlungsgeräts (4) umfasst, wobei ein zweiter Ventilator (6; 6A, 6B) zum Zuführen von Luft in die Umgebung des medizinischen Behandlungsgeräts (4) vorgesehen ist.

7. Medienversorgungseinrichtung (1) gemäß einem der vorstehenden Ansprüche, **dadurch gekennzeichnet, dass** die Medienversorgungseinrichtung (1) zum Versorgen von mindestens zwei medizinischen Behandlungsgeräten (4A, 4B) mit Medien eingerichtet ist und für jedes der medizinischen Behandlungsgeräte (4A, 4B) ein separater Zuluftkanal (5A, 5B) und/oder ein separater Abluftkanal (2A, 2B) vorgesehen ist.

8. Medienversorgungseinrichtung (1) gemäß einem der vorstehenden Ansprüche, **dadurch gekennzeichnet, dass** die Klimatisierungsvorrichtung (10) eine Drosselklappe (9) zum Mischen von Zuluft und Abluft aufweist.

9. Medienversorgungseinrichtung gemäß einem der vorstehenden Ansprüche, **dadurch gekennzeichnet, dass** die Klimatisierungsvorrichtung (10) mit dem medizinischen Behandlungsgerät direkt thermisch koppelbar ist.

10. Medienversorgungseinrichtung gemäß einem der vorstehenden Ansprüche, **dadurch gekennzeichnet, dass** die Klimatisierungsvorrichtung (10) einen Schalldämpfer (32, 62) aufweist, wobei der Schalldämpfer (32, 62) an einem Zuluftkanal (5) und/oder einem Abluftkanal (2) und/oder einem Ventilator (3, 6) angebracht ist.

11. Medienversorgungseinrichtung (1) gemäß einem der vorstehenden Ansprüche, **dadurch gekennzeichnet, dass** mindestens ein Luftkanal (5) der Klimatisierungsvorrichtung (10) ein Heizelement und/oder ein Kühlelement (76) und/oder ein Befeuchtungselement (78) und/oder ein Entfeuchtungselement aufweist, welches mit der Steuer-/Regeleinheit (7) kommunikativ verbunden ist, wobei die Steuer-/Regeleinheit (7) dazu eingerichtet ist, die Temperatur und/oder die Luftfeuchtigkeit des jeweiligen Luftstroms basierend auf dem jeweiligen Wert des Sensors zu steuern/regeln.

12. Medienversorgungseinrichtung (1) gemäß einem der vorstehenden Ansprüche, **dadurch gekennzeichnet, dass** die Klimatisierungsvorrichtung (10) über ein drahtloses Bedienelement einstellbar ist und die Klimatisierungsvorrichtung (10) dazu eingerichtet ist, einen Sollwert basierend auf einer Kennung eines Benutzers einzustellen.

13. Medienversorgungseinrichtung (1) gemäß Anspruch 12, **dadurch gekennzeichnet, dass** die Klimatisierungsvorrichtung (10) über Funk, WLAN, Bluetooth, Zigbee, NFC, Wi-Fi, RFID oder Infrarot einstellbar ist und wobei das Bedienelement (40) als Endgerät in Form eines Smartphones, Smartwatches oder Tablets ausgebildet ist.

14. Medienversorgungseinrichtung (1) gemäß einem der vorstehenden Ansprüche, **dadurch gekennzeichnet, dass** die Klimatisierungsvorrichtung (10) einen Klimatisierungsanschluss bereitstellt, an welcher das zu klimatisierende medizinische Behandlungsgerät anschließbar ist.

15. Medizinisches Behandlungssystem mit mindestens einem medizinischen Behandlungsgerät, **gekennzeichnet durch** eine Medienversorgungseinrichtung mit einer Klimatisierungsvorrichtung gemäß einem der vorstehenden Ansprüche.

## Claims

1. A media supply apparatus (1) for supplying a medical treatment device (4) with a medium,
**characterised by**
an air conditioning device (10) for air conditioning a separate medical treatment device (4) that is connected to the media supply apparatus (1), wherein the air conditioning device (10) comprises an exhaust air duct (2; 2A, 2B) for discharging air from the surroundings of the medical treatment device (4), wherein a first ventilator (3; 3A, 3B) for discharging air from the surroundings of the medical treatment device (4) into the exhaust air duct (2; 2A, 2B) is provided.

2. The media supply apparatus (1) according to claim 1, **characterised by** a connection (100) for connecting the medical treatment device (4) to feed a medium to the medical treatment device (4), and/or a drain connection (120) for discharging a medium from the medical treatment device (4).

3. The media supply apparatus (1) according to any one of the preceding claims, **characterised in that** the air conditioning device (10) has a temperature sensor (70; 70A, 70B) for measuring the temperature of the medical treatment device (4; 4A, 4B) and/or the ambient temperature of the medical treatment device (4; 4A, 4B) and a control unit (7) that is communicatively connected with the temperature sensor (70; 70A, 70B) and is configured to control the air conditioning performance of the air conditioning device (10) based on a value of the temperature sensor (70; 70A, 70B).

4. The media supply apparatus (1) according to claim 3, **characterised in that** the control unit (7) is configured to receive an operation parameter or an operating parameter that corresponds to a specific operating mode of the medical treatment device (4) and to control the air conditioning performance based on the operating parameter.

5. The media supply apparatus (1) according to claim 3 or 4, **characterised in that** the control unit (7) is configured to control the air conditioning performance via a volume flow of a respective ventilator connected to the control unit (7).

6. The media supply apparatus (1) according to any one of the preceding claims, **characterised in that** the air conditioning device (10) comprises a supply air duct (5; 5A, 5B) for supplying air into the surroundings of the medical treatment device (4), wherein a second ventilator (6; 6A, 6B) is provided for supplying air into the surroundings of the medical treatment device (4).

7. The media supply apparatus (1) according to any one of the preceding claims, **characterised in that** the media supply apparatus (1) is configured to supply at least two medical treatment devices (4A, 4B) with media and wherein a separate supply air duct (5A, 5B) and/or a separate exhaust air duct (2A, 2B) is provided for each of the medical treatment devices (4A, 4B).

8. The media supply apparatus (1) according to any one of the preceding claims, **characterised in that** the air conditioning device (10) has a throttle valve (9) for mixing supply air and exhaust air.

9. The media supply apparatus according to any one of the preceding claims, **characterised in that** the air conditioning device (10) is directly thermally couplable to the medical treatment device.

10. The media supply apparatus according to any one of the preceding claims, **characterised in that** the air conditioning device (10) has a silencer (32, 62), wherein the silencer (32, 62) is arranged at a supply air duct (5) and/or an exhaust air duct (2), and/or a ventilator (3, 6).

11. The media supply apparatus (1) according to any one of the preceding claims, **characterised in that** at least one air duct (5) of the air conditioning device (10) has a heating element and/or a cooling element (76) and/or a humidifying element (78) and/or a dehumidifying element which is communicatively connected to the control unit (7), wherein the control unit (7) is configured to control the temperature and/or the humidity of the respective air flow based on the respective value of the sensor.

12. The media supply apparatus (1) according to any one of the preceding claims, **characterised in that** the air conditioning device (10) is adjustable via a wireless operating element and the air conditioning device (10) is configured to set a target value based on an identifier of a user.

13. The media supply apparatus (1) according to claim 12, **characterised in that** the air conditioning device (10) is adjustable via radio, WLAN, Bluetooth, Zigbee, NFC, Wi-Fi, RFID or infrared, and wherein the operating element (40) is designed as a terminal device in the form of a smartphone, smartwatch or tablet.

14. The media supply apparatus (1) according to any one of the preceding claims, **characterised in that** the air conditioning device (10) provides an air conditioning connection to which the medical treatment device to be air conditioned is connectable.

15. A medical treatment system with at least one medical treatment device, **characterised by** a media supply apparatus with an air conditioning device according to any one of the preceding claims.

## Revendications

1. Appareil d'alimentation en milieux (1) pour alimenter un appareil de traitement médical (4) avec un milieu,
**caractérisé par**
un dispositif de climatisation (10) pour la climatisation d'un appareil de traitement médical (4) raccordé à l'appareil d'alimentation en milieux (1) et séparé, dans lequel le dispositif de climatisation (10) comprend un canal d'évacuation d'air (2 ; 2A, 2B) pour évacuer de l'air de l'environnement de l'appareil de traitement médical (4), dans lequel un premier ventilateur (3 ; 3A, 3B) est prévu pour évacuer de l'air de l'environnement de l'appareil de traitement médical (4) dans le canal d'évacuation d'air (2 ; 2A, 2B).

2. Appareil d'alimentation en milieux (1) selon la revendication 1, **caractérisé par** un raccord (100) pour raccorder l'appareil de traitement médical (4) pour amener un milieu à l'appareil de traitement médical (4) et/ou un raccord de sortie (120) pour évacuer un milieu de l'appareil de traitement médical (4).

3. Appareil d'alimentation en milieux (1) selon l'une quelconque des revendications précédentes, **caractérisé en ce que** le dispositif de climatisation (10) présente un capteur de température (70 ; 70A, 70B) pour mesurer la température de l'appareil de traitement médical (4; 4A, 4B) et/ou la température ambiante de l'appareil de traitement médical (4; 4A, 4B) et une unité de commande/régulation (7) connectée de manière communicative au capteur de température (70 ; 70A, 70B) est configurée pour commander/réguler la puissance de climatisation du dispositif de climatisation (10) sur la base d'une valeur du capteur de température (70 ; 70A, 70B).

4. Appareil d'alimentation en milieux (1) selon la revendication 3, **caractérisé en ce que** l'unité de commande/régulation (7) est configurée pour recevoir un paramètre de fonctionnement ou un paramètre de fonctionnement du dispositif de traitement médical (4) correspondant à un mode de fonctionnement déterminé et pour commander/réguler la puissance de climatisation sur la base du paramètre de fonctionnement.

5. Appareil d'alimentation en milieux (1) selon la revendication 3 ou 4, **caractérisé en ce que** l'unité de commande/régulation (7) est configurée pour commander/réguler la puissance de climatisation par l'intermédiaire d'un débit volumétrique d'un ventilateur respectif connecté à l'unité de commande/régulation (7).

6. Appareil d'alimentation en milieux (1) selon l'une quelconque des revendications précédentes, **caractérisé en ce que** le dispositif de climatisation (10) comprend un canal d'alimentation en air (5 ; 5A, 5B) pour amener de l'air dans l'environnement de l'appareil de traitement médical (4), dans lequel un second ventilateur (6 ; 6A, 6B) est prévu pour amener de l'air dans l'environnement de l'appareil de traitement médical (4).

7. Appareil d'alimentation en milieux (1) selon l'une quelconque des revendications précédentes, **caractérisé en ce que** l'appareil d'alimentation en milieux (1) est configuré pour alimenter en milieux au moins deux appareils de traitement médical (4A, 4B) et un canal d'alimentation en air séparé (5A, 5B) et/ou un canal d'évacuation d'air séparé (2A, 2B) est prévu pour chacun des appareils de traitement médical (4A, 4B).

8. Appareil d'alimentation en milieux (1) selon l'une quelconque des revendications précédentes, **caractérisé en ce que** le dispositif de climatisation (10) présente un papillon (9) pour mélanger de l'air d'alimentation et de l'air d'évacuation.

9. Appareil d'alimentation en milieux selon l'une quelconque des revendications précédentes, **caractérisé en ce que** le dispositif de climatisation (10) peut être couplé thermiquement directement à l'appareil de traitement médical.

10. Appareil d'alimentation en milieux selon l'une quelconque des revendications précédentes, **caractérisé en ce que** le dispositif de climatisation (10) présente un silencieux (32, 62), dans lequel le silencieux (32, 62) est attaché à un canal d'alimentation en air (5) et/ou un canal d'évacuation d'air (2) et/ou un ventilateur (3, 6).

11. Appareil d'alimentation en milieux (1) selon l'une quelconque des revendications précédentes, **caractérisé en ce qu'**au moins un canal d'air (5) du dispositif de climatisation (10) présente un élément de chauffage et/ou un élément de refroidissement (76) et/ou un élément d'humidification (78) et/ou un élément de déshumidification qui est connecté de manière communicative à l'unité de commande/régulation (7), dans lequel l'unité de commande/régulation (7) est configurée pour commander/réguler la température et/ou l'humidité du flux d'air respectif sur la base de la valeur respective du capteur.

12. Appareil d'alimentation en milieux (1) selon l'une quelconque des revendications précédentes, **caractérisé en ce que** le dispositif de climatisation (10) est réglable par l'intermédiaire d'un élément de commande sans fil, et le dispositif de climatisation (10) est configuré pour régler une valeur de consigne sur la base d'un identifiant d'un utilisateur.

13. Appareil d'alimentation en milieux (1) selon la revendication 12, **caractérisé en ce que** le dispositif de climatisation (10) est réglable par radio, WLAN, Bluetooth, Zigbee, NFC, Wi-Fi, RFID ou infrarouge et dans lequel l'élément de commande (40) est conçu en tant que terminal sous forme d'un smartphone, d'une smartwatch ou d'une tablette.

14. Appareil d'alimentation en milieux (1) selon l'une quelconque des revendications précédentes, **caractérisé en ce que** le dispositif de climatisation (10) fournit un raccord de climatisation auquel l'appareil de traitement médical à climatiser peut être raccordé.

15. Système de traitement médical avec au moins un appareil de traitement médical, **caractérisé par** un appareil d'alimentation en milieux avec un dispositif de climatisation selon l'une quelconque des revendications précédentes.
